# EUROPEAN PATENT SPECIFICATION

(11) **EP 3 911 339 B1**
(45) Date of publication and mention of the grant of the patent: **08.01.2025**
(21) Application number: 20846564.1
(22) Date of filing: 24.07.2020
(51) Int. Cl.: A61K 35/12, A61K 35/14, A61K 35/28

(54) **PLATELET RICH-FIBRIN DERIVED MESENCHYMAL STEM CELLS**
MESENCHYMALE STAMMZELLEN AUS BLUTPLÄTTCHENREICHEM FIBRIN
CELLULES SOUCHES MÉSENCHYMATEUSES DÉRIVÉES DE FIBRINE RICHE EN PLAQUETTES

(30) Priority: 30.07.2019 TR 201911506
(43) Date of publication of application: 24.11.2021
(73) Proprietor: T.C. Erciyes Üniversitesi, 38039 Kayseri (TR)
(72) Inventor: GÖNEN, Zeynep Burçin, Kayseri (TR); ÇETIN, Mustafa, Talas/Kayseri (TR); BAHAR, Dilek, Talas/Kayseri (TR); ÖZKAN, Buket Banu, Talas/Kayseri (TR); SALKIN, Hasan, Talas/Kayseri (TR)
(74) Representative: Yalçiner Patent and Consulting Ltd.
(86) International application number: PCT/TR2020/050647
(87) International publication number: WO 2021/021051

(56) References cited:
- WO-A1-2011/069121
- WO-A1-2014/053420
- KR-B1- 101 878 441
- US-A1- 2006 210 544
- I KASSIS ET AL: "Isolation of mesenchymal stem cells from G-CSF-mobilized human peripheral blood using fibrin microbeads", BONE MARROW TRANSPLANTATION, vol. 37, no. 10, 1 May 2006 (2006-05-01), pages 967 - 976, XP055044142, ISSN: 0268-3369, DOI: 10.1038/sj.bmt.1705358
- XU LIANGLIANG ET AL: "Circulating mesenchymal stem cells and their clinical implications", JOURNAL OF ORTHOPAEDIC TRANSLATION, vol. 2, no. 1, 15 December 2013 (2013-12-15), pages 1 - 7, XP055907660, ISSN: 2214-031X, Retrieved from the Internet <URL:http://dx.doi.org/10.1016/j.jot.2013.11.002> DOI: 10.1016/j.jot.2013.11.002
- DOHAN EHRENFEST D M ET AL: "Classification of platelet concentrates: from pure platelet-rich plasma (P-PRP) to leucocyte- and platelet-rich fibrin (L-PRF)", TRENDS IN BIOTECHNOLOGY, ELSEVIER PUBLICATIONS, CAMBRIDGE, GB, vol. 27, no. 3, 1 March 2009 (2009-03-01), pages 158 - 167, XP025958233, ISSN: 0167-7799, [retrieved on 20090131], DOI: 10.1016/J.TIBTECH.2008.11.009
- DOHAN ET AL: "Platelet-rich fibrin (PRF): A second-generation platelet concentrate. Part I: Technological concepts and evolution", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 101, no. 3, 1 March 2006 (2006-03-01), pages e37 - e44, XP005339143, ISSN: 1079-2104, DOI: 10.1016/J.TRIPLEO.2005.07.008
- DOHAN EHRENFEST DAVID M. ET AL: "Three-Dimensional Architecture and Cell Composition of a Choukroun's Platelet-Rich Fibrin Clot and Membrane", JOURNAL OF PERIODONTOLOGY., vol. 81, no. 4, 1 April 2010 (2010-04-01), US, pages 546 - 555, XP055907691, ISSN: 0022-3492, DOI: 10.1902/jop.2009.090531
- AB KADIR RUZANNA, ZAINAL ARIFFIN SHAHRUL HISHAM, MEGAT ABDUL WAHAB ROHAYA, KERMANI SHABNAM, SENAFI SAHIDAN: "Characterization of mononucleated human peripheral blood cells", THE SCIENTIFIC WORLD JOURNAL, vol. 2012, 12 January 2012 (2012-01-12), pages 843843, XP055792691, DOI: 10.1100/2012/843843

## Description

### Technical Field

The invention is related to the yield of Mesenchymal Stem Cell (MSC) from Platelet Rich-Fibrin (PRF) obtained from human blood in good manufacturing practice laboratory environment (GMP lab) in the therapeutic stem cell applications in medicine and health, its culturing and making it ready for use in the clinic as an advanced therapy products (ATMP).

### Prior Art

Stem cells are specialized cells that can self-replicate during cell division and differentiate into other cell types. Stem cells are classified as embryonic stem cells (ESC), induced pluripotent stem cells (I PSC) and mesenchymal stem cells (MSC).

MSCs are adult stem cells and can be obtained from humans and animals. MSCs are named in this way because they can differentiate into special cells that develop from the mesoderm. Human mesenchymal stem cells (hMSC) are multipotent cells that are self-regenerative, multipotent, adherent, easily obtainable, widely cultured under laboratory conditions, and can differentiate into mesodermal line cells such as osteocytes, adipocytes and chondrocytes.

The minimum criteria determined by the International Society for Cellular Therapy (ISCT) for the characterization of hMSC are listed as follows: These cells; a. Must adhere to the plastic surface, b. CD14, CD34, CD45 and HLA-DR markers should not express, while CD73, CD90 and CD105 are expressing, c. Must have an ability to differentiate *in vitro* into osteocytes, adipocytes and chondrocytes. These characteristics are applied to all MSCs. In the state of the art, hMSCs were first isolated from the bone marrow and then obtained from various tissue sources such as adipose tissue, amniotic fluid, amniotic membrane, limb bud (embryo limb bud) menstrual blood, peripheral blood, placenta and fetal membrane, salivary gland, skin and foreskin, synovial fluid endometrium, dental pulp, umbilical cord and Wharton jelly. hMSCs can be cultured in media specifically produced for themselves for the long-term. MSCs have been used in the treatment of various diseases for a long time with their immunomodulatory properties, the factors they secrete, the immunreceptors that they organize their own microenvironment, and their differentiation.

MSCs obtained from the placenta and the umbilical cord in the patent document numbered WO2009052132 and the ones obtained from wharton jelly in the patent document numbered US2018362923 are some the methods of obtaining only when there is a pregnancy.

In the state of the art, various invasive methods are employed to obtain hMSCs. Employing the invasive method to obtain hMSC is undesirable for the comfort of the subject. For example, while using MSCs taken from the bone marrow of the subject to obtain MSC from bone marrow; in MSCs to be obtained from adipose tissue, it is usually applied by removing fatty tissue pieces from the belly region with liposuction (fat removal method) or small operations. MSCs to be obtained from dental pulp must be obtained from deciduous teeth, however this can be only in childhood, it is not suitable for obtaining stem cells in adults. It is obtained from wisdom teeth or other teeth in later ages. However, it is not suitable for the comfort of the subject since this process requires an operation. MSCs to be obtained from tissues such as limb bud, placenta and fetal membrane, Wharton jelly and umbilical cord, amniotic fluid, amniotic membrane, foreskin are also not a source of stem cells which can be used in adult ages as they need to be taken at a certain time. Still, stem cell sources such as synovial fluid, endometrium and salivary gland are also sources of MSCs in which cells can be obtained by invasive methods. In the prior art, it has been demonstrated that obtaining MSC from peripheral blood is performed by obtaining cells with both hematopoietic and MSC characteristics from the blood by Ficoll gradient cell precipitation method. However, with the invention, peripheral whole blood is not used in obtaining MSC. Platelet Rich-fibrin (PRF) tissue is used for obtaining MSC. In the prior art, whole blood was used directly for obtaining MSC and it was shown that there is both hematopoietic and MSC stem cell in the blood. However, PRF is completely different from whole blood in terms of both content and structure. PRF is a fibrin matrix that mainly contains thrombocyte and cytokines. With the invention, the presence of MSC is also shown inside. The presence of MSC was reported in tissue containing many connective tissue stromas in the body. Blood is not an organ/tissue containing stroma. Although peripheral blood is required initially for obtaining PRF, PRF has a completely different anatomical, physiological and molecular structure from peripheral blood. Thus, there is no known prior art for obtaining MSC from PRF. In the prior art used for obtaining MSC from blood, which can be considered the closest, among the obtained cells, those with adherence properties were evaluated with CD105, KIT and SLAMF1 markers, the morphology of the cells was found acceptable, and their differentiation ability into the only osteoblast and osteoclast cells was examined. Therefore, since there was no study that fulfilled the criteria determined by the I SCT, the cells adhered to the plastic surface were evaluated as cells exhibiting the MSC character. Platelet Rich-Fibrin (PRF) refers to a fibrin matrix portion obtained by specific centrifugation technique of Peripheral blood. This portion is enriched in thrombocyte, is different from the content of peripheral blood in terms of both cellular and cytokines. Although the presence of CD34+ hematopoietic stem cells in Platelet Rich-Fibrin (PRF) is known in the state of the art, Platelet Rich-Fibrin (PRF) is not available for use for mesenchymal stem cell production.

### Summary and Purposes of The Invention

The primary aim of the invention is to obtain mesenchymal stem cells with blood taken directly from the venous vein without affecting the comfort of the subject without any invasive procedure.

With the invention, it is aimed to obtain thrombocyte-rich fibrin tissue and to obtain mesenchymal stem cells from this tissue by taking only 1 tube of blood from an easily obtainable source, without requiring any surgical operation.

With the invention, mesenchymal stem cells were obtained from thrombocyte-rich fibrin tissue, and the produced cells were also prepared in clinically usable production conditions (GMP grade) and made clinically applicable.

### Definitions of Drawings Illustrating the Invention

**Figure 1** **:**Morphological image of PRF/MSCs

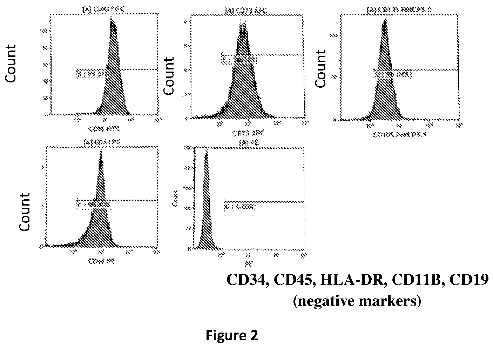

**Figure 2****:** The result of the characterization of cells with flow cytometry
**Figure 3****:** Oil globules in the adipogenically differentiated cells
**Figure 4****:** Microscopic image of cells which were differentiated by remaining in osteogenic medium for two weeks
**Figure 5****:** Microscopic image of cells which were differentiated by remaining in chondrogenic medium for two weeks

### Detailed Description of The Invention

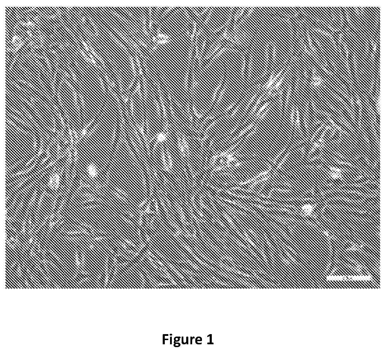

Platelet Rich-Fibrin (PRF) means fibrin matrix structure which is obtained from natural blood tissue, comprises abundant thrombocytes and leukocytes in its structure. With blood obtained from volunteers; Mesenchymal stem cells (MSC) were obtained from PRF (Figure 1). For this purpose, 8-50 mL of venous blood (preferably 10 mL) was firstly collected from each subject with a syringe and transferred into glass-covered plastic tubes. PRFs were obtained by centrifuging the tubes for 10 minutes at 3000 rpm according to a known method. Then, PRFs were washed with PBS. Under the sterile conditions, they were mechanically cut into small pieces as possible with a bistoury under the laminar flow cabinet. The obtained small pieces were firstly transferred to D-Lysine coated flasks and were cultured in incubator with 0.3- 5% CO₂ at 37°C by adding 8-20% by volume of human serum (preferably 12.5), animal serum or 0.5-2% by volume of penicillin-streptomycin (preferably 1%) without serum; 0.5-2% by volume of L-glutamine (preferably 1%); inorganic salts, such as calcium chloride, potassium chloride, magnesium sulfate, sodium chloride, sodium bicarbonate, sodium phosphate; amino acids, such as L-alanine, L-arginine, L-asparagine-H2O, L-aspartic acid, L-cystine, L-cysteine-HCl-H2O, L-glutamic acid, L-glutamine, glycine, L-histidine, L- isoleucine, L-leucine, L-lysine, L-methionine, L-phenyl alanine, L-proline, L-serine, L-threonine, L-tryptophan, L-tyrosine, L-valine; vitamins, such as L-ascorbic acid, biotin, D-Ca pantothenate, choline chloride, folic acid, i-inositol, niacinamide, pyridoxal HCl, riboflamine, thiamine HCl, vitamin B12; alpha MEM medium, which is a finished commercial medium containing all of D-glucose lipoic acid, sodium pyruvate, phenol red substances. One day later, the contents in the D-Lysine coated flask were transferred to the empty flasks. During the 3-4 days following the primary culture, cells were monitored under an inverted microscope without moving much, and then the medium was changed. The medium was changed at least twice a week by monitoring the cells regularly. In the passaging process, when the cells were 85-90% confluent, the cells were removed from the plastic surface to which they were adhered by using 0.01-0.5% by volume of Trypsin-EDTA solution (preferably 0.05%), and after an average of 5 minutes of centrifugation at 200-400g (preferably 300g) for 3-8 minutes, the pellet was plated in new culture plates with the new medium. Upon reaching the therapeutic dose, the cells were optionally taken into glass vials at 30 × 10⁶ (±5 × 10⁶) cell/ml in a physiological fluid, such as physiological saline at a dose determined according to the disease to be used, for example; in 14 ml of physiological saline or in a physiological fluid which can be administered intravenously. Clinically useful mesenchymal stem cells were made ready for use in orthopedic-skeletal system diseases including graft versus host disease, heart diseases, brain diseases, liver diseases, cancer, jaw diseases, kidney and urinary system diseases, gynecological diseases, infectious diseases, eye diseases, osteoarthritis, osteonecrosis which have MSC indications and all dermatological cases having an anti-aging purpose.

In the characterization of PRF-MSCs to be performed by flow cytometry, cells exhibiting phenotypic MSC characterization in vitro at the 3. passage were used. For this, 1×10⁶ cells were removed from the culture plates, centrifuged and incubated with BD Stem Flow hMSC kit (BD cat no: 562245) containing CD11b, CD19, CD34, CD44, CD45, CD73, CD90, CD105 and HLA-DR markers according to the manufacturer's instructions after resuspended in DPBS. Then, the presence and ratios of CD markers were detected by using Navios (Beckman Coulter, USA) flow cytometry. The obtained results were analyzed with KALUZA (Beckman Coulter, USA) program (Figure 1). The expressions of negative markers CD11b, CD19, CD45, CD34 and HLA-DR are 0,03%; the expression of positive CD105 surface marker is 96,04%; the expression of CD73 surface marker is 96,08%; the expression of CD90 surface marker is 99,57%; the expression of CD44 surface marker is 95,12%. According to the MSC criteria, negative surface markers must be equal to or less than 2% and positive surface markers must be equal to or higher than 95% in phenotyping. The obtained data meet the criteria.

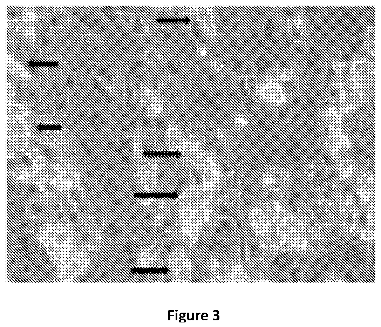

PRF/MSCs were plated at 3000 cells/cm² to 6-well plates to induce adipogenic differentiation at 3. passage. When the cells reached 90-100% confluency, the culture medium on the cells was aspirated and adipogenic differentiation of cells was induced by enculturation with an "adipogenic differentiation kit" (Gibco, USA) for 21 days. Differentiated cells were transformed into adipocytes containing round lipid droplets. Cell differentiation was monitored by staining oil droplets between the cells induced to differentiate adipogenically by AdipoRed Assay Kit (Lonza MD, USA). By staining, adipocytes were observed as pink oil droplets under an inverted microscope (Figure 3).

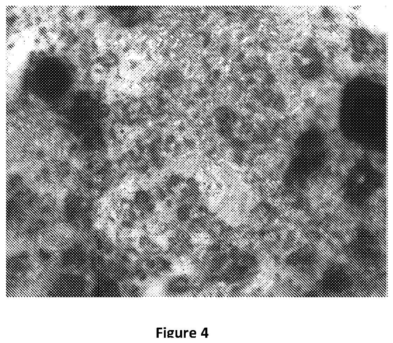

PRF-MSCs at 3. passage were seeded in 6-well plates at 3000 cells/cm² and when the cells reached 90-100% confluence, the culture medium was replaced with "osteogenic differentiation kit" (Biological Industries, Israel) and osteogenic differentiation of the cells was induced. The cells were induced for two weeks by changing the medium at least twice a week. At the end of two weeks, the calcium depots in the cells were stained with 1% Alizarin Red and the calcium depots in the osteogenically differentiated cells were observed under the inverted microscope by staining (Figure 4).

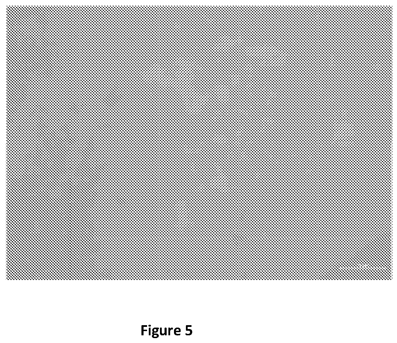

For chondrogenic differentiation, 500.000 PRF-MSCs cells at 3. passage were also taken in 15 ml Falcon tube and centrifuged at 300 g for 5 min. After centrifugation, the pellet was slowly replaced with a "chondrogenic differentiation kit" (Gibco, USA) without removing the pellet by discarding the supernatant. The transformation of the cells into chondrocyte was induced for two weeks by changing the medium at least twice a week. Cells that differentiated at the end of two weeks were examined by staining with a human mesenchymal stem cell functional identification kit (R&D System, USA) (Figure 5). For this purpose, the cell pellet differentiated in a three-dimensional falcon tube was frozen by placing on the frozen secting compound (cat no: 3801480, Leica) solution dropped over the cryomold and sectioned by cryostat method. Freezing and sectioning processes were performed in the Leica CM1860 UV device. Subsequently, agregante proteoglycans found in extracellular matrix structure were displayed under a fluorescence microscope by staining with Agrecan Antigen Affinity-purified Polyclonal Antibody. Differentiated cells were imaged with a fluorescence microscope.

## Claims

1. Method of obtaining mesenchymal stem cells, comprising the steps of:
• Obtaining Platelet Rich-Fibrin (PRF) by a centrifugation method from blood taken from a venous blood,
• Cutting Platelet rich-fibrin (PRF) tissue into pieces under a laminar flow cabinet,
• Transferring the obtained PRF pieces firstly into D-Lysine coated flasks and adding thereto 8-20% by volume of human serum, animal serum or 0.5-2% by volume of penicillin-streptomycin without serum; 0.5-2% by volume of L-glutamine; inorganic salts, such as calcium chloride, potassium chloride, magnesium sulfate, sodium chloride, sodium bicarbonate, sodium phosphate; amino acids, such as L-alanine, L-arginine, L-asparagine-H2O, L-aspartic acid, L-cystine, L-cysteine-HCl-H2O, L-glutamic acid, L-glutamine, glycine, L-histidine, L- isoleucine, L-leucine, L-lysine, L-methionine, L-phenyl alanine, L-proline, L-serine, L-threonine, L-tryptophan, L-tyrosine, L-valine; vitamins, such as L-ascorbic acid, biotin, D-Capantothenate, choline chloride, folic acid, i-inositol, niacinamide, pyridoxal HCl, riboflavin, thiamine HCl, vitamin B12; alpha MEM medium containing all of D-glucose lipoic acid, sodium pyruvate, phenol red substances,
• Culturing in an incubator containing 0,3-5% by volume of COz at 37°C,
• Removing of stem cells from the surface to which they were adhered by using 0,01-0,5% by volume of Trypsin-EDTA solution and after 3-8 minutes of centrifugation at 200-400g,
• Obtaining PRF-derived Mesenchymal stem cells (PRF-Derived MSCs).

2. The method of obtaining mesenchymal stem cells according to claim 1, comprising the steps of;
• Re-culturing of the MSCs in medium for obtaining the cell expansion,
• Mesenchymal stem cells are **characterized by** flow cytometry before therapeutic administration,
• Upon reaching the therapeutic dose, cells are placed in plates at 30 × 10⁶ (±5 × 10⁶) cells/mL in physiological saline or in a physiological fluid that can be administered intravenously at a dose determined according to the disease.

## Patentansprüche

1. Verfahren zur Erzielung von mesenchymalen Stammzellen, umfassend die folgenden Schritte:
• Erzielung von Plättchen reichem Fibrin (PRF) durch ein Zentrifugationsverfahren aus Blut, das einem venösen Blut entnommen wurde,
• Schneiden von Plättchen reichem Fibrin (PRF) gewebe in Stücke unter einer Laminar-Flow-Kabine,
• Überführung der erhaltenen PRF-Stücke zunächst in mit D-Lysin beschichtete Kolben und Zugabe von 8-20 Vol.- % Humanserum, tierischem Serum oder 0.5-2 Vol.- % Penicilin-Streptomycin ohne Serum; 0.5-2 Vol.- % L-Glutamin; anorganische Salze, wie Calciumchlorid, Kaliumchlorid, Magnesiumsulfat, Natriumchlorid, Natriumbicarbonat, Natriumphosphat; Aminosäuren, wie L-Alanin, L-Arginin, L-Asparagin-H20, L- Asparaginsäure, L-Cystin, L-Cystin- HCl -H20, L-Glutaminsäure, L- Glutamin, Glycin, L-Histidin, L- Isoleucin, L- Leucin, L-Lysin, L-Methionin, L-Phenylalin, L- Prolin, L-Serin, L-Threonin, L-Tryptophan, L-Tyrosin, L-Valin; Vitamine, wie L-Ascorbinsäure, Biotin, D-Capantothenat, Cholinchlorid, Folsäure, i-Inositol, Niacinamid, Pyridoxal HCl, Riboflavin, Thiamin-HCl, Vitamin B12; Alpha MEM-Medium, das die gesamte D-Glucose enthält, Liponsäure, Natriumpyruvat, Phenolrote Stoffe,
• Kultivierung in einem Inkubator mit 0,3-5 Vol.- % CO₂ bei 37°C,
• Entfernen von Stammzellen von der Oberfläche, an der sie haften, unter Verwendung von 0,01-0,5 Vol.- % Trypsin-EDTA-Lösung und nach 3-8 Minuten Zentrifugation bei 200-400 g,
• Erzielung von PRF-abgeleiteten Mesenchymalen Stammzellen (PRF-abgeleitete MSCs)

2. Verfahren zur Erzielung von mesenchymalen Stammzellen nach Anspruch 1, umfassend die folgenden Schritte:
• Rekultivierung der MSCs in Medium zur Erzielung der Zellexpansion,
• Mesenchymale Stammzellen werden vor therapeutischer Verabreichung durch Durchflusszytometrie charakterisiert,
• Nach Erreichen der therapeutischen Dosis werden die Zellen in Platten mit 30 × 10⁶ (±5 × 10⁶) zellen/mL in physiologischer Kochsalzlösung oder in einer physiologischen Flüssigkeit platziert, die intravenös in einer je nach Krankheit bestimmten Dosis verabreicht werden kann.

## Revendications

1. Méthode d'obtention de cellules souches mésenchymateuses, comprenant les étapes suivantes :
• Obtenir la Fibrine Riche en Plaquettes (PRF) par une méthode de centrifugation à partir de sang prélevé dans un sang veineux,
• Couper le tissu de Fibrine Riche en Plaquettes (PRF) en morceaux sous une armoire à flux laminaire,
• Transférer les morceaux de PRF obtenus tout d'abord dans des flacons enduits de D-Lysine et y ajouter 8-20% en volume de sérum humain, de sérum animal ou 0,5-2% en volume de pénicilline-streptomycine sans sérum ; 0,5-2% en volume de L-glutamine ; sels inorganiques, tels que chlorure de calcium, chlorure de potassium, sulfate de magnésium, chlorure de sodium, bicarbonate de sodium, phosphate de sodium ; les acides aminés, tels que la L-alanine, la L-arginine, la L-asparagine-H2O, l'acide L-aspartique, la L-cystine, la L-cystéine-HCl-H₂O, l'acide L-glutamique, la L-glutamine, la glycine, L-histidine, L-isoleucine, L-leucine, L-lysine, L-méthionine, L-phénylalanine, L-proline, L-sérine, L-thréonine, L-tryptophane, L-tyrosine, L-valine ; vitamines, telles que acide L-ascorbique, biotine, D-Capantothénate, chlorure de choline, acide folique, i-inositol, niacinamide, pyridoxal HCl, riboflavine, thiamine HCl, vitamine B12 ; milieu alpha MEM contenant toutes les substances de D-glucose, de l'acide lipoïque, du pyruvate de sodium, du rouge de phénol,
• Culturer dans un incubateur contenant 0,3-5% en volume de CO₂ à 37°C,
• Retirer les cellules souches de la surface à laquelle elles ont adhéré en utilisant 0,01-0,5% en volume de solution de Trypsine-EDTA et après 3-8 minutes de centrifugation à 200-400 g,
• Obtenir les cellules souches mésenchymateuses dérivées du PRF (MSC dérivées du PRF).

2. Méthode d'obtention de cellules souches mésenchymateuses selon la revendication 1, comprenant les étapes suivantes ;
• Reculturer les MSC dans un milieu pour obtenir l'expansion cellulaire,
• Les cellules souches mésenchymateuses sont **caractérisées par** cytométrie de flux avant l'administration thérapeutique,
• Après avoir atteint la dose thérapeutique, les cellules sont placées dans des plaques à 30 × 10⁶ (±5 × 10⁶) cellules/mL dans une saline physiologique ou dans un fluide physiologique qui peut être administré par voie intraveineuse à une dose déterminée en fonction de la maladie.
